# EUROPEAN PATENT SPECIFICATION

(11) **EP 1 687 023 B1**
(45) Date of publication and mention of the grant of the patent: **03.10.2012**
(21) Application number: 04799038.7
(22) Date of filing: 04.11.2004
(51) Int. Cl.: A61K 39/008, A61K 39/015, G01N 33/569, A61P 33/02

(54) **USE OF SPECIFIC HISTONES FOR THE TREATMENT OF PARASITIC DISEASES**
VERWENDUNG VON SPEZIFISCHEN HISTONEN ZUR BEHANDLUNG VON PARASITISCHEN ERKRANKUNGEN
APPLICATION D'HISTONES SPECIFIQUES AU TRAITEMENT DE MALADIES PARASITAIRES

(30) Priority: 05.11.2003 WO PCT/EP03/12421; 23.02.2004 WO PCT/EP2004/001782
(43) Date of publication of application: 09.08.2006
(73) Proprietor: C.B.F. Leti, S.L. Unipersonal, 28760 Tres Cantos-Madrid (ES)
(72) Inventor: ALVAREZ, Manuel, Soto, E-28915 Madrid (ES); IBORRA, Salvador, E-28053 Madrid (ES); REQUENA, José, Maria, E-08038 Barcelona (ES); BEDATE, Carlos, Alonso, E-200015 Madrid (ES)
(74) Representative: van Westenbrugge, Andries
(86) International application number: PCT/IB2004/004078
(87) International publication number: WO 2005/044301

(56) References cited:
- WO-A-97/33999
- US-A1- 2003 138 451
- MELBY P C ET AL: "Identification of vaccine candidates for experimental visceral leishmaniasis by immunization with sequential fractions of a cDNA expression library" INFECTION AND IMMUNITY, AMERICAN SOCIETY FOR MICROBIOLOGY. WASHINGTON, US, vol. 68, no. 10, October 2000 (2000-10), pages 5595-5602, XP002284060 ISSN: 0019-9567
- SOTO M ET AL: "Characterization of the antigenic determinants of the Leishmania infantum histone H3 recognized by antibodies elicited during canine visceral leishmaniasis" CLINICAL AND EXPERIMENTAL IMMUNOLOGY, vol. 106, no. 3, 1996, pages 454-461, XP008045786 ISSN: 0009-9104
- SOTO M ET AL: "ANTIGENICITY OF THE LEISHMANIA INFANTUM HISTONES H2B AND H4 DURING CANINE VISCEROCUTANEOUS LEISHMANIASIS" CLINICAL AND EXPERIMENTAL IMMUNOLOGY, OXFORD, GB, vol. 2, no. 115, February 1999 (1999-02), pages 342-349, XP001086535 ISSN: 0009-9104
- SOTO MANUEL ET AL: "Multicomponent chimeric antigen for serodiagnosis of canine visceral leishmaniasis" JOURNAL OF CLINICAL MICROBIOLOGY, vol. 36, no. 1, January 1998 (1998-01), pages 58-63, XP002137284 ISSN: 0095-1137
- REQUENA J M ET AL: "Immune and clinical parameters associated with Leishmania infantum infection in the golden hamster model" VETERINARY IMMUNOLOGY AND IMMUNOPATHOLOGY, AMSTERDAM, NL, vol. 76, no. 3-4, 31 October 2000 (2000-10-31), pages 269-281, XP002284059 ISSN: 0165-2427
- IBORRA S ET AL: "Vaccination with a plasmid DNA cocktail encoding the nucleosomal histones of Leishmania confers protection against murine cutaneous leishmaniosis" VACCINE, BUTTERWORTH SCIENTIFIC. GUILDFORD, GB, vol. 22, no. 29-30, 28 September 2004 (2004-09-28), pages 3865-3876, XP004567462 ISSN: 0264-410X

### SEQUENCE LISTING

<110> C.B.F. Leti S.L., Unipersonal
<120> Use of specific histones for the treatment of parasitic diseases
<130> P211394PCT
<160> 8
<170> PatentIn version 3.1
<210> 1
   <211> 25
   <212> DNA
   <213> Leishmania infantum
<400> 1
   cgggatccat ggtactcctc gcagc 25
<210> 2
   <211> 27
   <212> DNA
   <213> Leishmania infantum
<400> 2
   cccaagctta cgcgctcggt gtcgccc 27
<210> 3
   <211> 25
   <212> DNA
   <213> Leishmania infantum
<400> 3
   cgggatccgc ctcttctcgc tctgc 25
<210> 4
   <211> 29
   <212> DNA
   <213> Leishmania infantum
<400> 4
   cccaagcttc aagccgacgc gctcgacac 29
<210> 5
   <211> 27
   <212> DNA
   <213> Leishmania infantum
<400> 5
   cgggatccat gtcccgcacc aaggaga 27
<210> 6
   <211> 29
   <212> DNA
   <213> Leishmania infantum
<400> 6
   cctaagcttc tagtggcgct caccgcgca 29
<210> 7
   <211> 27
   <212> DNA
   <213> Leishmania infantum
<400> 7
   cgggatccat ggccaagggc aagcgtt 27
<210> 8
   <211> 27
   <212> DNA
   <213> Leishmania infantum
<400> 8
   cccaagctta cgcgtagccg tacagga 27

## Description

### Field of the invention

The present invention relates to the use of histones for the treatment, or prevention of a Leishmaniasis disease. In particular, it relates to the use of all four histone proteins H2A, H2B, H3 and H4 for such uses, wherein the Leishmaniasis disease is caused by a different Leishmania species than the species from which the histones were derived.

### Background of the invention

*Leishmania* protozoa are obligate intracellular parasites that infect cells of the mononuclear phagocyte lineage of their vertebrate hosts. These parasites are the etiological agents of leishmaniases, a group of diseases characterized by a variety of clinical manifestations in humans, ranging from self-healing cutaneous ulcers to potentially fatal visceral infection (Herwardt, Lancet (1999) 354:1191). The development of the disease and the spread of the infection vary greatly from individual to individual depending on the genetic background and the status of the immune system of the infected host

Murine models of cutaneous leishmaniasis have been extensively used to explore the requirements for effective vaccination. The administration of several antigens to mice encoded in DNA vaccines, results in the prevention of the disease in some cases (reviewed in Handman, Clin. MicrobioL Rev. (2001) 14:229. Vaccinations with DNA encoding the proteins GP63, LACK and PSA-2 protected mice from *L*. *major*-infection (see for instance Xu et al, Immunology (1955) 84:173. Furthermore, an immunization with a combination of plasmids expressing the proteins LACK, LmST11 and TSA has been proven to be highly effective against low dose challenge with *L. major* (Mendez et al. J. ImmunoL (2001) 166: 5122, whereas a cocktail of two plasmids encoding the cysteine proteinases CPa and CPb partially protected BALB/c mice against high dose challenge with *L. major* (Rafati et al, Vaccine (2001) 19 (25-26): 3369). Most of the protection experiments are restricted at the laboratory level and with animal models. Thus, since there are scarce field studies in real host animals there is no definitive proof that the candidates will be successful vaccines.

It should be noted that an antigen which produces a good antibody titer, i.e. which is immunogenic, is not always and per se offering protection against a disease. On the contrary, while some antibody or cellular responses against an antigen may be associated with protection, other antibody or cellular responses against the antigen may be associated with susceptibility and/or exacerbation of the disease. In addition, an adjuvant is often necessary for protective effect, since antigens without adjuvant may produce an antibody response, but not offer protection. See for instance, Stacey and Blackwell (Infect Immun. 1999 Aug,67(8):3719-26) who reported that subcutaneous vaccination of mice with soluble Leishmania antigen (SLA) alone, SLA plus alum, or SLA plus non-oligodeoxynucleotides (ODN) which contain immunostimulatory CG motifs (CpG ODN) resulted in exacerbated disease compared to unvaccinated mice. Mice receiving SLA plus CpG ODN showed a highly significant protective effect compared to SLA-vaccinated mice and enhanced survival compared to unvaccinated mice.

The antigenic diversity among *Leishmania* species, implying species-specific epitopes, may be one pitfall in the development of a vaccine based on individual antigens (Handman, 2001, vide supra). Known DNA vaccines only protect against the species from which the DNA was obtained. Melby and colleagues reported in Infect Immun. (2000) 68:5595 that immunization with a cDNA expression library and sub-libraries coding for *Leishmania donovani* proteins protected mice against the VL form of the disease caused by *L. donovani.* Gurunathan et al., J. Exp. Med. (1977) 186: 1137 reported that vaccination with DNA encoding the immunodominant LACK (leishmania homologue of receptors for activated C kinase) parasite antigen from *L. major* confers protection against *Leishmania major.* Melby et al. Infect Immun. (2001) 69:4719 show that this antigen does not confer protection against *L. infantum* in mice. They also report that although *L. donovani* p36 (LACK) DNA vaccine is highly immunogenic, it is not protective against ex-perimental visceral leishmaniasis. This phenomenon that an immunogenic molecule does not confer protection i.e. does not lead to clearance of an infection or to release of clinical symptoms, is one of the challenges of finding a good vaccine. If an immunogenic or antigenic molecule would always automatically give protection, finding a vaccine would be easy. Finding a vaccine which can be used against more than one species is even more of a challenge.

Melby P.C. et al (Melby P.C. et al, Infection and Immunity, American Society for Microbiology, Washington, US, Vol 68, no.10, October 2000, pages 5595-5602) describes the use of *L. donovani* H2B, H3 and H4 histones for protection against *L*. *donovani.*

Soto M. et al (Soto M. et al, J. Of Clin. Microbiol., (1998), vol 36: 58-63) describe the use of a recombinant chimeric protein, namely PQ containing fragments of H2A as a vaccine.

US 2003/138451 describes the use of a chimeric polypeptide comprising H2A with other antigen as a vaccine.

### Detailed description

The present invention relates to the use of all four histone proteins H2A, H2B H3 and H4 for the treatment, or prevention of a Leishmaniasis disease, wherein the Leishmaniasis disease is caused by a different Leishmania species than the species from which the histones were derived.

The advantage of the present invention is that it allows for the preparation of a medicament for the treatment of a broader spectrum of diseases i.e. a medicament with cross-species specifity. In many parasitic diseases, as Leishmaniasis a vaccine raised against a specific species, only works against that specific species. At the moment, the disease is controlled by drugs, but drug treatment does not prevent the spread of the disease and in many cases is not very effective.

In one aspect of the invention, the histones are used for the preparation of a pharmaceutical composition for the treatment of a Leishmaniasis disease, wherein the Leishmaniasis disease is caused by a different Leishmania species than the species from which the histones were derived.

In one embodiment of the invention, in this pharmaceutical composition, each histone is present in equimolar amounts.

The pharmaceutical composition of the invention may be used to increase the ability of the human or animal immune system to fight infections. In particular, it may be used for administration to a human or animal subject. The pharmaceutical composition is preferably administered parenterally, e.g. by injection or infusion by intravenous, intraperitoneal, intramuscular, intraarterial or intralesional route. The pharmaceutical composition may be combined with a pharmaceutically acceptable medium or delivery vehicle by conventional techniques known in the art. Methods for preparing parenterally administrable compositions are well known in the art and described in more detail in various sources, including, for example, Remington's Pharmaceutical Sciences, Ed. AR Gennaro, 20th edition, 2000, Williams & Wilkins, PA, USA. The pharmaceutical composition is preferably administered in a therapeutically effective dose, i.e. one that will increase the ability of the human or animal immune system to fight infections.

Of particular interest is the pharmaceutical composition that is a vaccine. The vaccine may be a protein based vaccine.

The vaccine may also be a DNA vaccine. The DNA vaccine comprises a gene for each histone which is present in the vaccine. In this application, such genes encoding more than one histone are called chimeric genes.

The genes in a DNA vaccine according to the present invention will typically be present in vectors. Examples of suitable vectors are well known to the person skilled in the art, see for instance Donnelly et al, Ann. Rev. Immunol. (1997) 15: 617 and include but are not limited to pcDNA3 and pcCMV.

The DNA in the vaccine may be any DNA which is suitable for protein expression, for instance cDNA or dsDNA but not genomic DNA or ssDNA. There may also be a mixture of protein and DNA in one vaccine by simultaneous or successive administration of the DNA and protein. Also RNA vaccines may be used, although these will need stabilizers to stabiles the RNA which is to be administered.

There are over 20 known species of Leishmania, including species of the subgenus Leishmania, comprising the complex *L. major,* including *L. major,* the complex *L*. *Donovani*, including *L*. *chagasi*, *L. donovani* and *L. infantum*, the complex *L. Mexicana*, including *L. amazonensis* and *L. mexicana*, as well as the subspecies Viannia, comprising the complex *L. braziliensis*, including *L. braziliensis* and *L. peruviana* and the complex *L. guyanensis*, including *L. guyanensis* and *L. panamensis*. *Plasmodium* species of particular interest are *Plasmodium falciparum* and *Plasmodium vivax.*

- In one embodiment of the invention, a pharmaceutical composition comprising histones from one species is used for the preparation of a medicament for the treatment of a Leishmaniasis disease caused by another species.

In particular, *Leishmaniasis* caused by one species from the genus Leishmania may be treated by using a pharmaceutical composition based on histones from another *Leishmania* species. In one embodiment, *Leishmania* caused by *L*. *major* is successfully treated with a composition comprising histones from *L*. *infantum*.

In another aspect, the invention provides for a method to prevent Leishmaniasis infection by functioning as a therapeutic vaccine. Typically, there is a time period between infection and disease. In this case the vaccine would act as a pharmacological immune product that would cure the disease by eliciting in the host an immune response that counteracts the pathological effect of the infection. A therapeutic vaccine differs from a prophylactic vaccine in that a therapeutic vaccine will induce protection in a patient who already has the infection or the disease.

### Short description of the figures

**Fig. 1**.
(A) Protection in mice vaccinated with three doses of histone gene pools. BALB/c mice inoculated with Leishmania histone-genes and control (pcDNA3 or PBS) were challenged with 5 × 10⁴ *L. major* promastigotes injected in the left hind footpad. Footpad swelling is given as the difference between the thickness of the infected versus the contralateral uninfected footpad. Weekly footpad measurements represent the average foot pad scores ± SD. *L.major* challenge in DNA immunized mice is representative of two different experiments with virtually the same results. *At the fourth week the differences between the footpad swelling of controls and vaccinated mice were significant (*P* < 0.001).
**(B)** Parasite loading in spleen and lymph nodes eight weeks after infection in controls (pcDNA3) and ten weeks in vaccinated (pcDNA3-histones). Cell suspensions were made from the popliteal lymph node of infected leg and the spleen. The number of viable parasites was determined by limiting dilution. Results are expressed as mean ± SD of the total number of parasites per tissue (expressed as decimal logarithm). *Differences between controls and vaccinated mice were significant in both spleen and popliteal lymph nodes (*P* < 0.001).

### EXAMPLES

### Example 1

### Production of a DNA vaccine comprising histones from L. infantum

To determine whether DNA vaccines encoding *Leishmania* histones were able to protect mice against *L. major* infection, *L. infantum* H2A, H2B, H3 and H4 genes were subcloned into the eukaryotic expression vector pcDNA3 for expression under the control of a strong cytomegalovirus (CMV) promoter. Then, expression of *Leishmania* histones was assessed in COS7 cells transfected with the pcDNA3 constructs. Expression of the recombinant proteins by mammalian cells transfected with plasmid DNA is a critical condition for the stimulation of the immune system. Transfected COS7 cells were cultured during 3 days and the expression of the *Leishmania* histones was assessed by Western blot analyses.

### 1.1 Cloning of cDNA in the mammalian expression vector pCDNA3

For the expression of the four core histones of *Leishmania infantum* in the pCDNA3 mammalian expression vector four full length cDNA clones were employed: H2A (clone cL72; Soto et al, Eur.J. Biochem. 1992, 205(1):211-6); H2B (clone LiH2B; Soto et al. , Clin. Exp. Immunol., 1999, 115(2):342-9); H3 (clone LiB6; Soto et al., Biochim. Biophys. Acta, 1994, 18:1219(2):533-5) and H4 (clone LiH4-1; Soto et al., Clin. Exp. Immunol. 1999, 115(2):342-9). All of these cDNA clones were previously isolated from a *L*. *infantum λ*gt11 cDNA expression library. The EcoRI inserts from the cDNA clones were subcloned in the corresponding cut site of the pCDNA3 mammalian expression vector. DNA form those pcDNA3 recombinant plasmids were purified by the endotoxin-free Giga-preparation Kit (Qiagen, Hilden, Germany).

### 1.2 Expression of Leishmania histones in mammalian cells.

To confirm that the DNA construct was functional and expressed die mentioned proteins, COS7 cells were transfected with 20 µg of each pcDNA3 construct prepared in section 1.1 using the Lipofectin® Reagent (Gibco, BRL) according to the manufacturer's protocol Briefly, 3 × 10⁶ cells were seeded on 100 mm plates in Dulbecco's modified Eagle's medium plus 5% FCS and transfected when they reached 50 to 75% confluence. Seventy-two hours post-transfection, the cells were harvested, washed two times with ice-cold PBS and immediately lysed by addition of Laemmli's buffer. Protein derived from equivalent numbers of cells were resolved by sodium dodecyl sulphate-polyacrylamide gel electrophoresis (SDS-PAGE) and transferred to nitrocellulose membranes (Amersham, Aylesbury, UK). The blots were probed with the sera from dogs suffering from visceral Leishmaniasis (VCL) and purified by affinity chromatography against H2B, H3, H4 proteins in the immobilized phase) or rabbit immunized with rH2A.

The results showed that transfected cells expressed detectable levels of H2A, H2B, H3 and H4 proteins.

### 1.3 Cloning into pQE30 expressing vector

For expression of the *L infantum* histones as recombinant proteins, the coding regions were PCR-amplified taking as template the pcDNA3 constructs from 1.1 and the following nucleotides as primers:
For the H2A gene (sense 5'-CGGGATCCATGGTACTCCTCGCAGC-3' (positions 1-17 of the coding region); antisense 5'-CCCAAGCTTACGCGCTCGGTGTCGCCC-3' (reverse and complementary to positions of the coding region);
For the H2B gene (sense 5'-CGGGATCCGCCTCTTCTCGCTCTGC-3' (positions 1-17 of the coding region);. antisense 5'-CCCAAGCTTCAAGCCGACGCGCTCGACAC-3' (reverse and complementary to positions of the coding region);
For the H3 gene (sense 5'-CGGGATCCATGTCCCGCACCAAGGAGA-3' (positions 1-19 of the coding region); antisense 5'-CCCAAGCTTCTAGTGGCGCTCACCGCGCA-3' (reverse and complementary to positions of the coding region); and
For the H4 gene (sense 5'-CGGGATCCATGGCCAAGGGCAAGCGTT-3' (positions 1-19 of the coding region); antisense 5'-CCCAAGCTTACGCGTAGCCGTACAGGA-3' (reverse and complementary to positions of the coding region).

The underlined nucleotides represent the BamHI and the HindIII cut sites included to allow the cloning of the PCR products in the pQE30 expression vector (Qiagen, Hilden, Germany). The resulting clones were named pQE-H2A, pQE-H2B, pQE-H3 and pQE-H4.

### 1.4 Expression and purification of the His-tagged recombinant proteins

Expression and purification of the His-tagged recombinant proteins was performed in *Escherichia coli* M15 following standard procedures (Qiagen). After induction of the protein, the bacteria were harvested, and lysed by sonication under denaturing conditions (8 M urea, 0.5 M NaCl, 20 mM Tris-HCl): The purification of the protein was performed on a NI-NTA agarose column (Qiagen). Recombinants proteins were gradually refolded on the affinity column as described (Shi, 1997). Afterwards, the recombinant protein was eluted with 0.3 M imidazol. After elution, fractions containing the recombinant histones were pooled and dialyzed against PBS. The purified protein was further passed through a polymixin-agarose column (Sigma, St Louis, MO) in order to eliminate endotoxins. Residual endotoxin was measured with Quantitative Chromogenic *Limulus* Amebocyte assay (QCL-1000, BioWhittaker, Walkersville, MD).

### Example 2 Immunogenetic properties of the DNA vaccine

### 2.1 Preparation of total Leishmania antigen (SLA).

Soluble *Leishmania* antigen (SLA) was prepared by three freezing and thawing cycles of stationary promastigotes of *L. major* resuspended in PBS. After cell lysis, soluble antigens were separated from the insoluble fraction by centrifugation.

### 2.2 Immunizations and parasite challenge.

Immunization experiments were carried out in groups of ten mice. Mice were inoculated three limes intramuscularly (i.m.) at 2-week intervals in both hind leg quadriceps with 50 µg (25 µg per leg) of each plasmid DNA (pcDNA3-H2A, pcDNA3-H2B, pcDNA3-H3 and pcDNA3-H4) as prepared in section 1.1 in a total volume of 100 µl of PBS. Control mice were inoculated following the same schedule but with 200 µg of pcDNA3 in each inoculation or PBS alone. Fourteen days after every inoculation mice were bled by orbital plexus puncture. Four weeks after the final inoculation, spleens and lymph nodes (LN) from immunized mice were collected and groups of five immunized mice were challenged with cultured 5 × 10⁴ stationary phase promastigotes injected in the left hind footpad. The footpad swelling was measured every week and is given as the difference between the thickness of the infected footpad versus contralateral control footpad.

### 2.3 Determination of antibody titres and isotypes.

Serum samples were analyzed for specific anti-*Leishmania* histones antibodies. Briefly, standard ELISA plates were coated overnight at room temperature with 100 µl of rH2A, rH2B, rH3 and rH4 (1 µg/ml in PBS) or SLA (2 µg/ml in PBS). A serial dilution of the sera was carried out in order to determine the titer, which is defined as the inverse of the highest serum dilution factor giving an absorbance > 0.2. The isotype-specific analyses were done with the following horseradish peroxidase-conjugated anti-mouse immunoglobulins (Nordic Immunological Laboratories, Tilburg, The Netherlands): anti-IgG1 (1:1000) and anti-IgG2a (1:500). Ortophenyle diamine dihydrochloride -OPD- (Dako, A/S, Glostrup, Denmark) was used as peroxidase substrate. After 15 min, the reaction was stopped with the addition of 100 □l of H₂SO₄ 1 M, and the absorbance was read at 450 nm.

### 2.4 Measurement of cytokines in supernatants.

The release of IFN-δ and IL-4 was measured in the supernatants of splenocytes and LNCs cultures. Spleens and lymph nodes from BALB/c mice were removed aseptically after cervical dislocation. Splenocyte and lymph node cells suspensions were seeded in complete RPMI medium (RPMI 1640 supplemented with 10% FCS, 2mM glutamine, and 10mM 2-mercaptoethanol). 3 x 10⁶ cells were seeded in 48-well plates during 72 h at 37°C in the presence of rH2A, rH2B, rH3 and rH4 (12 µg/ml), histones from calf thymus (SIGMA) or Con A (2 □g/ml). The cytokine production was determined by commercial ELISA kits (Diaclone, Besançon, France).

### 2.5 Parasite quantitation.

The number of parasites was determined in popliteal lymph node from infected leg and spleen by limiting dilution (Buffet, et al., 1995). The tissues were homogenized and serially diluted in a 96-well flat-bottomed microtiter plate containing Schneider's medium plus 20% FCS. The number of viable parasites per lymph node was determined from the highest dilution at which promastigotes could be grown up to 7 days incubation at 26°C.

### 2.6 Statistical analysis.

Statistical analysis was performed by a Student's *t*-test. Differences were considered significant when p < 0.05.

### 2.7 Antibody response after vaccination with the DNA vaccine

To study the immunogenic properties of the cDNAs, BALB/c mice were immunized intramuscularly two or three times every two weeks with a mixture of the four plasmids prepared in section 1.1 encoding the *Leishmania* infantum histones (50 µg of cDNA each). Antibodies specific for these antigens were evaluated 2 weeks after the last immunization. No antibodies specific were detected by ELISA after two inoculations whereas IgG antibodies became detectable at low titers after the third inoculation IgG2a was the predominant isotype of antibodies in all cases. Only a slight production of IgG1 antibodies was also detected against the H2A and H4 histones.

### 2.8 T-cell response after vaccination with the DNA vaccine

To measure the T cell response after vaccination splenic mononuclear cells and lymph node cells (LNCs) were obtained 4 weeks after the last DNA immunization and were stimulated in vitro with the recombinant proteins prepared in section 1.4. After 3 days of incubation, the supernatants were harvested and assayed for both IFN-δ and IL-4.

It was observed that in immunized mice the recombinant histone proteins stimulated the production of IFN-δ in an amount that is larger than that found in controls in both splenocytes and LNCs cells . No IL-4 histone-specific production could be detected in the supernatants of any of the cultures.

### 2.9 Analysis of the IL-12 dependence

In order to characterize in more detail the immune response elicited in mice by DNA vaccination with the *L. infantum* histone genes and its evolution after challenging with *L. major,* the antigen-specific production of IFN-γ by splenocytes from vaccinated mice was assessed. Twenty-five days after *L. major* infection, splenocytes from vaccinated mice continued to produce more histone-specific IFN-γ than splenocytes from control mice. Interestingly, the production of SLA-mediated IFN-γ was 2-fold higher in splenocytes from vaccinated mice as compared with that from controls. As IL-12-dependent production of IFN-γ is the main mechanism associated with the control of *L. major* infection, it was assessed whether addition of anti-IL-12 monoclonal antibody to cultures substantially inhibited production of IFN-γ in splenocytes from vaccinated mice. The inhibition of IFN-γ production was found to be higher than 80% for the four different stimuli used (*L. infantum* histones H2A, H2B, H3 and H4). This indicates that the histone-specific IFN-γ production is IL-12-dependent and, furthermore, that the in vitro production of IL-12 is also stimulated in splenocytes by the leishmanial histones.

### 2.10 The involvement of CD4+ and CD8+ T cells in the production of IFN-γ.

The relative contributions of CD4+ and CD8+ T cells to the production of IFN-γ was determined. The histone-mediated stimulation of IFN-γ production from splenocytes of all groups was substantially inhibited by addition to the cultures of anti-CD4 monoclonal antibodies. However, addition of anti-CD8 monoclonal antibodies to the cultures induced a reduction of IFN-γ secretion only in the splenocytes of mice vaccinated and in vitro stimulated with either histone H2A or H3. Thus, DNA vaccination with genes encoding *Leishmania* histones H2A and H3 seems to be priming more CD8 cells than the other two histone genes (H2B and H4).

Finally, in order to provide further insights into the immune status associated with the protection elicited in mice by DNA vaccination with the genes coding for the *L. infantum* histones, the frequency of CD4+ and CD8+ T cells producing IFN-γ in the LNCs was assessed by intracellular cytokine staining at the end of the protection studies (8 control mice and 10 vaccinated mice). The frequency of both CD4+ and CD8+ cells producing IFN-γ was higher in vaccinated mice than in controls. Thus, these data are a direct support that protection against *L. major* infection, achieved by DNA vaccination with *Leishmania* histones, is correlated in the vaccinated mice with an enhanced frequency of T cells (CD4+ and CD8+) producing IFN-γ.

These results are in agreement with the predominance of IgG2a antibodies in the sera of the immunized mice and indicate that DNA immunization with the H2A, H2B, H3 and H4 histone genes elicited preferentially a Th1-like immune response against these antigens. Further test experiments (see Example 3) had to show whether the genes also had a protective effect.

### Example 3 Protection against Leishmania major induced by the DNA vaccines encoding for Leishmania infantum histones.

Since the immunogenicity experiments revealed that DNA immunization with histones triggered the induction of specific Th1-like immune responses we next asked the question of whether the cDNA immunogenic regime using the H2A, H2B, H3 and H4 genes could provide protection against infection of mice with *L*. *major.*

### 3.1 DNA vaccine encoding histones from L. infantum protect against infection by L. major.

Groups of seven BALB/c mice were inoculated intramuscularly two or three times as described above, two weeks apart, with 50 µg of each histone-DNA construct as prepared in section 1.1. Controls were inoculated in the same way with 200 µg of the empty vector used for the cDNA cloning of the histones genes or with PBS alone. Four weeks after the last DNA immunization, the mice were challenged in the left footpad with cultured 5×10⁴ stationary promastigotes forms of *L. major* as prepared in section 2.1. Footpad swelling was measured weekly.

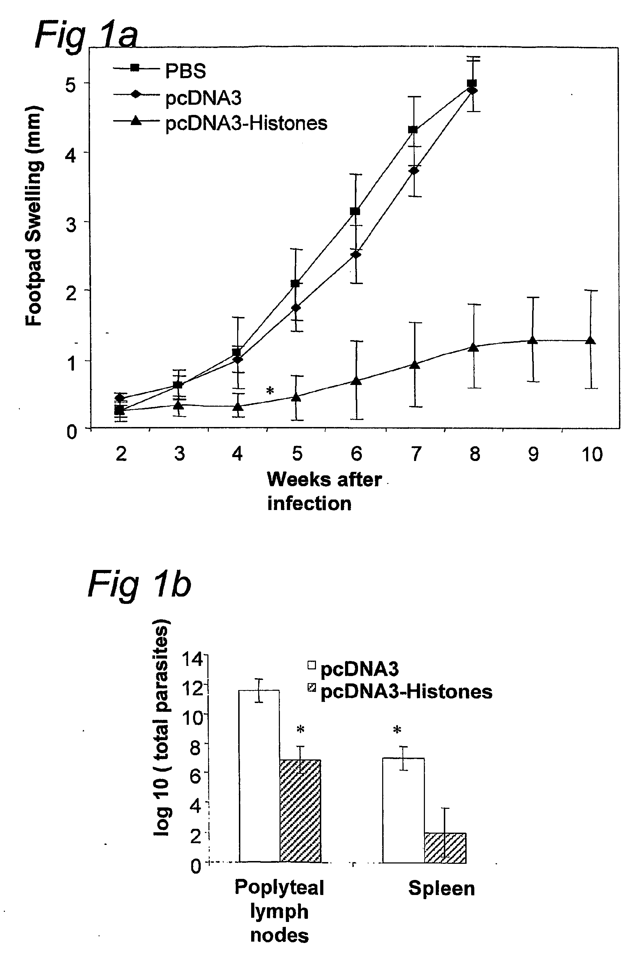

The results shown in Fig. 1A indicate that DNA vaccination with histones genes induced protection against infection, whereas vector alone did not. We have observed a clear delay in the footpad swelling in vaccinated mice. In all cases, immunized mice show a more controlled inflammation (about 1 mm at eight week after infection) than controls (about five mm at the same week). No lesions whatsoever were observed in four of seven mice during the course of infection. This experiment has been reproduced with similar results.

### 3.2 Parasite load of spleen and lymph node after vaccination with DNA vaccine encoding histones.

To determine whether the reduced footpad swelling was correlated with the parasite load in spleen and popliteal lymph nodes, mice were euthanized 10 weeks after infection of the vaccinated mice group and 8 weeks after infection of the controls). The vaccinated mice exhibited a significant lower parasite load than that determined in controls (Fig 1B). Remarkably, visceralization of the parasite was homogeneously observed in controls (pcDNA3) whereas in vaccinated mice the parasite load was low, and in some cases no parasites were detected. All these data suggest that vaccinated mice are controlling the cutaneous leishmaniosis induced by *L. major.*

### Example 4 Immunization with histones in an individual form.

The previous examples have shown that injection with a DNA cocktail of eukaryotic expression plasmids pcDNA3 containing the genes for the *Leishmania infantum* histones (H2A, H2B, H3 and H4) in BALB/c mice, leads to an specific Th1 immune response that results in the control of the *L. major* infection. In the present example, we studied the effect of the genetic immunization with each one of these pcDNA3 encoding the nucleosomal H2A, H2B, H3 and H4, administered separately. Experimental conditions were as described above.

The results indicate that in all cases the administration of each histone pcDNA3 induces a decrease of both IL-4 and IL-10 Th2 cytokines 9 weeks after the *L.major* infection concomitantly with a lower humoral response against soluble leishmanial antigen SLA and a limited humoral response against histones (dominated by antibodies IgG2a), compared with control mice immunized with an empty pcDNA3. In addition, results from proliferation analysis of splenocytes in the control mice show the existence of a depressed cellular immune response to the mitogens Con A, LPS and to SLA. During the course of infection, vaccinated mice exhibited a delay in footpad swelling and a lower parasite burden in the popliteal lymph nodes compared with control mice. In conclusion, the administration of each histone pcDNA3 encoding the nucleosomal histones of *Leishmania* decreases the Th2-driven response induced by *L. major* infection, leading to a low IL-4 and IL-10 production in the spleen, and a low humoral response that is correlated with protection against murine cutaneous leishmaniosis. The results also indicate that, among *Leishmania* histones, DNA immunization with H4 is the most effective in inducing protective immunity against cutaneous leishmaniosis.

## Claims

1. Use of all four *Leishmania* histones H2A, H2B, H3 and H4 for the preparation of a medicament for the treatment or prevention of a Leishmaniasis disease, wherein the Leishmaniasis disease is caused by a different *Leishmania* species than the species from which the histones were derived.

2. Use according to claims 1, wherein each histone is present in equimolar amounts.

3. Use according to claim 1 or 2 wherein the medicament is a vaccine.

4. Use according to claim 3 wherein the vaccine is a protein based vaccine.

5. Use according to claim 3 wherein the vaccine is a DNA vaccine which comprises the genes which encode at least one of the histones H2A, H2B, H3 and H4.

6. Use according to claim 5, wherein one or more genes of the histones H2A, H2B, H3 and H4 are inserted in one or more vectors.

7. Use according to claims 5 or 6 wherein the vaccine comprises a chimeric gene which encodes two or more histones.

8. Use of a vector comprising the nucleotides encoding the *Leishmania* histones H2A, H2B, H3 and H4 for the preparation of a medicament for the treatment or prevention of a Leishmaniasis disease, wherein the Leishmaniasis disease is caused by a different *Leishmania* species than the species from which the histones were derived.

9. Use of antibodies directed against all of the *Leishmania* histones H2A, H2B, H3 and H4 for the preparation of a medicament for the treatment or prevention of a Leishmaniasis disease, wherein the Leishmaniasis disease is caused by a different *Leishmania* species than the species from which the histones were derived.

10. Use according to claims 1-9 wherein the histones are obtained from *Leishmania infantum.*

11. Use asccording to claims 1-10 wherein the Leishmaniasis disease is caused by *L*. *major* or *L. infantum* species.

12. A pharmaceutical composition comprising all of the *Leishmania* histones H2A, H2B, H3 and H4 or genes encoding these histones in addition to a pharmaceutically acceptable adjuvant and/or carrier.

## Patentansprüche

1. Verwendung aller vier *Leishmania-Histone* H2A, H2B, H3 und H4 zur Herstellung eines Medikaments für die Behandlung oder Prävention einer Leishmaniose-Erkrankung, wobei die Leishmaniose-Erkrankung durch eine andere *Leishmania*-Art als die Art, aus der die Histone stammen, hervorgerufen wird.

2. Verwendung nach Anspruch 1, wobei jedes Histon in äquimolären Mengen vorliegt.

3. Verwendung nach Anspruch 1 oder 2, wobei das Medikament ein Impfstoff ist.

4. Verwendung nach Anspruch 3, wobei der Impfstoff ein auf einem Protein basierender Impfstoff ist.

5. Verwendung nach Anspruch 3, wobei der Impfstoff ein DNA-Impfstoff ist, der die Gene umfasst, die mindestens eines der Histone H2A, H2B, H3 und H4 codieren.

6. Verwendung nach Anspruch 5, wobei ein oder mehrere Gene der Histone H2A, H2B, H3 und H4 in einen oder mehrere Vektoren insertiert sind.

7. Verwendung nach Anspruch 5 oder 6, wobei der Impfstoff ein chimäres Gen umfasst, das zwei oder mehr Histone codiert.

8. Verwendung eines Vektors, der die Nucleotide umfasst, die die *Leishmania-*Histone H2A, H2B, H3 und H4 codieren, zur Herstellung eines Medikaments für die Behandlung oder Prävention einer Leishmaniose-Erkrankung, wobei die Leishmaniose-Erkrankung durch eine andere *Leishmania*-Art als die Art, aus der die Histone stammen, hervorgerufen wird.

9. Verwendung von Antikörpern, die gegen alle der *Leishmania*-Histone H2A, H2B, H3 und H4 gerichtet sind, zur Herstellung eines Medikaments für die Behandlung oder Prävention einer Leishmaniose-Erkrankung, wobei die Leishmaniose-Erkrankung durch eine andere *Leishmania*-Art als die Art, aus der die Histone stammen, hervorgerufen wird.

10. Verwendung nach den Ansprüchen 1 bis 9, wobei die Histone aus *Leishmania infantum* erhalten werden.

11. Verwendung nach den Ansprüchen 1 bis 10, wobei die Leishmaniose-Erkrankung durch die Art *L. majoroder L. infantum* hervorgerufen wird.

12. Arzneimittel, das alle der *Leishmania*-Histone H2A, H2B, H3 und H4 oder der Gene, die diese Histone codieren, zusätzlich zu einem pharmazeutisch verträglichen Adjuvans und/oder Träger umfasst.

## Revendications

1. Utilisation de l'ensemble des quatre histones H2A, H2B, H3 et H4 de *Leishmania* pour la préparation d'un médicament pour le traitement ou la prévention d'une Leishmaniose, dans laquelle la Leishmaniose est causée par une espèce de *Leishmania* différente de l'espèce dont sont dérivées les histones.

2. Utilisation selon la revendication 1, dans laquelle chaque histone est présente en quantités équimolaires.

3. Utilisation selon la revendication 1 ou 2, dans laquelle le médicament est un vaccin.

4. Utilisation selon la revendication 3, dans laquelle le vaccin est un vaccin à base protéique.

5. Utilisation selon la revendication 3, dans laquelle le vaccin est un vaccin à base d'ADN qui comprend les gènes qui codent au moins une des histones H2A, H2B, H3 et H4.

6. Utilisation selon la revendication 5, dans laquelle un ou plusieurs gènes des histones H2A, H2B, H3 et H4 sont insérés dans un ou plusieurs vecteurs.

7. Utilisation selon les revendications 5 ou 6, dans laquelle le vaccin comprend un gène chimère qui code deux ou plusieurs histones.

8. Utilisation d'un vecteur comprenant les nucléotides codant les histones H2A, H2B, H3 et H4 de *Leishmania* pour la préparation d'un médicament pour le traitement ou la prévention d'une Leishmaniose, dans laquelle la Leishmaniose est causée par une espèce de *Leishmania* différente de l'espèce dont sont dérivées les histones.

9. Utilisation d'anticorps dirigés contre l'ensemble des quatre histones H2A, H2B, H3 et H4 de *Leishmania* pour la préparation d'un médicament pour le traitement ou la prévention d'une Leishmaniose, dans laquelle la Leishmaniose est causée par une espèce de *Leishmania* différente de l'espèce dont sont dérivées les histones.

10. Utilisation selon les revendications 1 à 9, dans laquelle les histones sont obtenues à partir de *Leishmania infantum.*

11. Utilisation selon les revendications 1 à 10, dans laquelle la Leishmaniose est causée par l'espèce *L. major* ou *L. infantum.*

12. Composition pharmaceutique comprenant l'ensemble des quatre histones H2A, H2B, H3 et H4 de *Leishmania* ou des gènes codant ces histones associés à un adjuvant et/ou un support pharmacologiquement acceptable.
